# EUROPÄISCHE PATENTSCHRIFT

(11) **EP 3 448 327 B1**
(45) Veröffentlichungstag und Bekanntmachung des Hinweises auf die Patenterteilung: **26.07.2023**
(21) Anmeldenummer: 17719272.1
(22) Anmeldetag: 26.04.2017
(51) Int. Cl.: A61F 5/01

(54) **ORTHESE**
ORTHOSIS
ORTHÈSE

(30) Priorität: 29.04.2016 DE 102016108049
(43) Veröffentlichungstag der Anmeldung: 06.03.2019
(73) Patentinhaber: Ottobock SE & Co. KGaA, 37115 Duderstadt (DE)
(72) Erfinder: SIEWERT, Gordon, 37083 Göttingen (DE); KRUCHEM, Tim, 37073 Göttingen (DE)
(74) Vertreter: Gramm, Lins & Partner Patent- und Rechtsanwälte PartGmbB
(86) Internationale Anmeldenummer: PCT/EP2017/059894
(87) Internationale Veröffentlichungsnummer: WO 2017/186776

(56) Entgegenhaltungen:
- EP-A1- 1 334 704
- FR-A- 961 981
- FR-A1- 2 766 359
- US-A- 4 510 927
- US-A1- 2009 247 922
- US-A1- 2013 060 180

## Beschreibung

Die Erfindung betrifft eine Orthese mit einer Fußplatte zur Auflage eines Fußes, mit einer davon in proximaler Richtung abstehenden Fußstrebe und einer an der Fußstrebe befestigten Unterschenkelschiene, die über ein Gelenk um eine Gelenkachse relativ zu der Fußstrebe schwenkbar daran gelagert ist.

Orthesen sind orthopädietechnische Einrichtungen, die an dem Körper getragen werden, beispielsweise an einer Gliedmaße oder an dem Rumpf und über Befestigungselemente wie Gurte, Manschetten, Schuhe oder Klammern an dem jeweiligen Körperteil befestigt werden. Die Befestigungselemente können beispielsweise über Schnallen oder Klettverschlüsse verschlossen werden, um eine individuell an den Orthesenträger angepasste Festlegung an der jeweiligen Gliedmaße zu erreichen. Orthesen dienen grundsätzlich zur Unterstützung, Stabilisierung, Entlastung, Abstützung oder auch Bewegungseinschränkung der jeweiligen Gliedmaße oder Gelenke, ebenso zur Korrektur von Fehlstellungen, zur korrekten Ausrichtung oder Beibehaltung einer bestimmten Orientierung sowie zum Schutz von Gelenken und Gliedma-ßen.

Im Bereich der unteren Extremitäten sind Orthesen beispielsweise als Knie-Knöchel-Fuß-Orthesen (KAFO) oder als Knöchel-Fuß-Orthesen (AFO) ausgebildet und dienen beispielsweise zur Verringerung der Auswirkungen einer Schwächung oder eines Ausfalls der Fußhebermuskulatur. Dazu kann ein federbelastetes Gelenk im Bereich des natürlichen Knöchelgelenkes vorgesehen sein, die die Fußplatte gegenüber der Unterschenkelschiene vorspannt, um in der Schwungphase eine Dorsalflexion durchzuführen, so das weiterhin ein Durchschwingen des Fußes möglich ist, ohne dass die Zehen oder Fußspitze auf dem Boden schleift. Statt eines federbelasteten Gelenkes kann auch eine Feder eine Fußplatte mit der Unterschenkelschiene verbinden.

Aus der EP 2 563 300 B1, die den nächstliegenden Stand der Technik darstellt, ist eine Orthese zur Korrektur einer Beinfehlstellung bekannt, mit einem Auflageschenkel, der einen Fuß einer Person untergreift und einen Kontakt mit einer Lauffläche, also entweder einer Sohle oder einem Fußboden herstellt. Die Orthese weist eine seitlich nach oben ragende Schienenanordnung auf, die mit einer Befestigungseinrichtung mit dem Unterschenkel der Person verbindbar ist, wobei ein bei Belastung starres Winkelstück am Übergang von dem Auflageschenkel zur Schienenanordnung ausgebildet ist. Die Schienenanordnung ist durch ein etwa in Höhe des Kompromissdrehpunktes angeordnetes Drehgelenk in einen zur seitlichen Anlage an dem Fuß vorgesehenen Anlageschenkel und in eine zur seitlichen Anlage an dem Unterschenkel vorgesehene Schiene unterteilt. Die Schiene ist als federndes Element ausgebildet, über ein aus einer eingestellten Vorspannung des federnden Elementes relativ zu dem Unterschenkel resultierendes Drehmoment wird eine seitlich wirkende Korrekturkraft auf den Unterschenkel ausgeübt.

Die FR 2 766 539 A1 betrifft eine Orthese mit einem Fußteil und einem gelenkig daran gelagerten Unterschenkelteil. Das Fußteil umgibt den Fuß und kann an dessen Oberseite über einen Riemen verschlossen werden. Gelenkbereiche sind medial und lateral neben dem Knöchelgelenk ausgebildet.

Die FR 961 981 A betrifft eine Orthese mit einem Fußbügel und einer Unterschenkelschiene, die gelenkig aneinander gelagert sind. Der Fußbügel erstreckt sich seitlich bis neben das natürliche Knöchelgelenk, die Unterschenkelschiene wird über eine Manschette an dem Unterschenkel befestigt.

Die US 2013/060180 A1 betrifft eine orthopädische Trainingseinrichtung zur Anwendung im Knöchelbereich mit einem Fußteil und zumindest einer vertikalen Schiene, die lösbar an dem Fußteil befestigt ist. Die vertikale Schiene weist einen Unterschenkelteil und einen Fußabschnitt auf, der mit dem Fußteil verbindbar ist.

Die EP 1 334 704 A1 betrifft eine Knöchelorthese mit zwei seitlich entlang des Unterschenkels angeordneten Schienen, die gelenkig an einer angenähert u-förmigen Sohlenplatte befestigt sind. Die Sohlenplatte weist zwei nach oben abstehende Abschnitte auf, an deren oberen Ende die Unterschenkelschienen gelenkig gelagert sind. Auf Höhe des natürlichen Knöchelgelenkes sind die Abschnitte nach außen abstehend geformt.

Die US 2009/0247922 A1 betrifft eine Orthese mit einem schalenartigen Fußteil und zwei gelenkig daran gelagerten Unterschenkelschienen, die über ein Gurtsystem und eine an den Schienen befestigte Manschette an dem Nutzer festlegbar ist.

Die US 4 510 927 A betrifft eine Knöchelgelenksschiene mit einem u-förmigen Fußteil und zwei gelenkig daran medial und lateral angeordneten Unterschenkelschienen. Das Fußteil und die Unterschenkelschienen werden über Gurte an dem Fuß beziehungsweise dem Unterschenkel festgelegt.

Aufgabe der vorliegenden Erfindung ist es, eine Orthese bereitzustellen, die einen erhöhten Tragekomfort sowie eine verbesserte Führung des Fußteils und eine optimierte Führung einer Unterschenkelschiene ermöglicht.

Erfindungsgemäß wird diese Aufgabe durch eine Orthese mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die Orthese mit einer Fußplatte zur Auflage eines Fußes, mit einer davon in proximaler Richtung abstehenden Fußstrebe und einer an der Fußstrebe befestigten Unterschenkelschiene, die über ein Gelenk um eine Gelenksachse relativ zu der Fußstrebe schwenkbar daran gelagert ist, sieht vor, dass an dem proximalen Endbereich der Fußstrebe ein Absatz in die von der Fußplatte abgewandten Richtung angeordnet oder ausgebildet ist. Die Fußstrebe erstreckt sich möglichst nah an der knöchernen Struktur des Fußes anliegend von der Fußplatte in proximale Richtung, also nach oben in Richtung auf den Unterschenkel, so dass sich einerseits die Fußplatte an der Fußunterseite und andererseits die Fußstrebe an der seitlichen Struktur des Fußes anlegen oder in geringer Entfernung dazu daran entlanggeführt werden kann. Dadurch ergibt sich ein sehr schmaler Aufbau in medial-lateral-Richtung und die Orthese kann mit minimaler Beeinträchtigung in einem Schuh getragen werden. Um das Gelenk möglichst nahe in demjenigen Bereich positionieren zu können, in dem sich das natürliche Knöchelgelenk befindet, ist es vorteilhaft, wenn die Fußstrebe an ihrem proximalen Endbereich einen Absatz aufweist, der in Richtung von den Fuß weg gerichtet ist, also in der von der Fußplatte abgewandten Richtung angeordnet oder ausgebildet ist. Bei einer medialen Anordnung der Fußstrebe ist der Absatz in medialer Richtung orientiert, bei einer lateralen Anordnung der Fußstrebe ist der Absatz in lateraler Richtung orientiert. Der Absatz wird bei einer medialen Anordnung der Fußstrebe durch eine Kröpfung in medialer Richtung in der Fußstrebe und bei einer lateralen Anordnung der Fußstrebe durch eine Kröpfung in lateraler Richtung der Fußstrebe erzeugt. Dadurch wird dem Umstand Rechnung getragen, dass das Schienbein und das Wadenbein im Bereich des Sprunggelenkes Knöchelvorsprünge ausbilden, die eine geradlinige Fortführung der Fußstrebe erschweren oder unkomfortabel werden lassen. Durch den Absatz wird gewährleistet, dass das Gelenk und damit auch die Gelenkachse im Bereich des Knöchelvorsprunges und möglichst nahe an dem Knöchelvorsprung angeordnet sein können, ohne die Bewegung zu stören und den Aufbau in Medialrichtung zu vergrößern. Bevorzugt hat der Absatz eine Tiefe zwischen 5 mm und 15 mm und erstreckt sich über die gesamte Breite der Fußstrebe. Die Fußstrebe erstreckt sich möglichst geradlinig von der Fußplatte in proximale Richtung, wobei der Absatz unterhalb des jeweiligen Knöchelvorsprunges beginnt und sich von dem Fuß weg erstreckt, um dann wieder in proximaler Richtung eine Aufnahme für das Gelenk oder die Gelenkeinrichtung auszubilden. Die Aufnahme kann als gerader Abschnitt mit Befestigungseinrichtungen für die Gelenkeinrichtung ausgebildet sein.

Die Erfindung sieht vor, dass der proximale Endbereich und die Gelenkachse um die Längserstreckung der Fußstrebe tordiert orientiert sind, so dass bei einer flächigen Ausgestaltung der unteren Fußstrebe im Bereich der Anlenkung an die Fußplatte die Gelenkachse nicht mehr senkrecht zu der Fußstrebe orientiert ist, sondern bei einer Draufsicht in Längserstreckung der Fußstrebe um eine Winkel verdreht ist. Die Verdrehrichtung oder Tordierrichtung ist dabei in Richtung einer Innenrotation, so dass die Schwenkrichtung des Gelenks in einem spitzen Winkel zu einer Fußlängsachse oder einer Mittellinie der Fußplatte verläuft. Bei einer Orthese für einen rechten Fuß und einer medialen Anordnung der Fußstrebe ist die Gelenkachse in Draufsicht auf die Fußstrebe entgegen dem Uhrzeigersinn verdreht orientiert, bei einer Orthese für einen linken Fuß ist sie bei medialer Anordnung der Fußstrebe im Uhrzeigersinn verdreht oder tordiert orientiert.

Eine Weiterbildung der Erfindung sieht vor, dass der Absatz durch eine Kröpfung in der Fußstrebe oder einen an der Fußstrebe befestigten Winkel ausgebildet ist. Bei einer einstückigen Ausgestaltung der Fußstrebe wird der Absatz durch mehrfaches Umbiegen der Fußstrebe erhalten. Das Umbiegen oder die Kröpfung kann geradlinig entlang von Biege- oder Knicklinien oder mit vergrößerten Biegeradien erfolgen, so dass eine abgeknickte oder kurvenförmige Biegung erfolgt.

Alternativ zu einer einstückigen Ausgestaltung der Fußstrebe zumindest von der Fußplatte bis zum proximalen Ende oder Endbereich kann an der Fußstrebe ein Winkel befestigt sein, der eine entsprechende Kröpfung aufweist oder ausbildet, so dass ein modularer Aufbau der Orthese erreicht werden kann. An dem Winkel kann eine Aufnahmeeinrichtung für die Fußstrebe angeordnet oder ausgebildet sein, so dass unterschiedliche Winkel auf ein Grundmodell einer Fußstrebe bzw. einer Fußplatte mit einer daran angeordneten oder ausgebildeten Fußstrebe aufgesetzt werden können. Über die Winkel kann eine Individualisierung der jeweiligen Orthese leicht erfolgen. Unterschiedliche Kröpfungswinkel, Kröpfungsorientierungen sowie Absätze können innerhalb des Winkels leicht hergestellt werden, so dass eine Anpassung an unterschiedliche Einsatzzwecke sowie an unterschiedliche Patienten leicht erfolgen kann. Werden stabilere Gelenke eingesetzt oder sind unterstützende Federeinrichtungen notwendig, können größere Absätze vorgesehen sein, ist nur eine Führungsschiene notwendig, kann ein schmaler Absatz gewählt werden, so dass der Aufbau in medialer Richtung minimiert werden kann.

Die Fußstrebe und/oder der Winkel sind bevorzugt aus einem starren Material ausgebildet, um ein ausreichend großes Moment zwischen der Fußplatte und der Fußstrebe übertragen zu können. Neben Metall kann zur Herstellung der Fußstrebe und/oder des Winkels ein faserverstärktes Material verwendet werden. Sofern eine plastische Verformbarkeit des verwendeten Materials gegeben ist, kann dadurch bei gleichzeitiger ausreichender Starrheit oder Steifigkeit eine zusätzliche Anpassbarkeit der Orthese an den jeweiligen Nutzer beispielsweise durch einen Orthopädiemechaniker bewerkstelligt werden. Das Material ist so gewählt, dass nach der plastischen Verformung eine ausreichende Stabilität bereitgestellt wird, um eine sichere Führung in der Frontalebene und der Sagittalebene bereitzustellen und darüber hinaus eine ausreichende Torsionssteifigkeit um die Längsachse der Fußstrebe zu gewährleisten. Die Festigkeiten und Steifigkeiten sind so gewählt, dass bei einer Belastung ein gleichbleibender Winkel oder zumindest ein im Wesentlichen gleichbleibender Winkel zwischen der Fußplatte und der Fußstrebe sichergestellt ist. Das Material und dessen Dimensionierung muss geeignet sein, um ein gewünschtes Moment zwischen der Fußplatte und der Fußstrebe bzw. Unterschenkelschiene übertragen zu können.

Die Fußstrebe kann sich zumindest in ihrem distalen Bereich, der sich an die Fußplatte anschließt, in einem Winkel zwischen 80° und 100° von der Fußplatte in proximaler Richtung erstrecken, um eine möglichst gleichmäßige und nahe Anlage oder Parallelorientierung zu der knöchernen Struktur und der Weichteilstruktur des Fußes ermöglichen zu können.

Die Fußstrebe und gegebenenfalls der Winkel können aus einem Flachmaterial ausgebildet sein, wodurch eine leichte Anpassbarkeit sowohl der Orientierung als auch der Dimensionierung des Absatzes durch entsprechendes Verformen erleichtert wird.

Die Gelenkachse des Gelenkes, das zwischen der Unterschenkelschiene und der Fußstrebe angeordnet ist und die beiden Komponenten miteinander verbindet, kann in medial-lateral-Richtung, also vom Innenknöchel zum Außenknöchel in distaler Richtung einen geneigten Winkel aufweisen, das heißt, dass die Gelenkachse sich von der medialen Seite schräg nach unten erstreckt, also zur Horizontalen nach lateral abwärts geneigt ist. Eine solche Orientierung der Gelenkachse entspricht der natürlichen Gelenkachse des Knöchels, so dass eine verbesserte Führung des Fußes durch Nachfolgen der natürlichen Gelenkachse gegeben ist.

Darüber hinaus ist es möglich, dass die Gelenkachse in einem Winkel α schräg zu der Fußmittellinie verläuft, wobei die Knöchelgelenkachse bei einer medialen Anlage des Gelenkes an dem Fuß und dem Knöchel in posteriorer Richtung orientiert ist. Der Winkel α kann zwischen 70 ° und 89 ° betragen. Bei einer angenommenen Orientierung der natürlichen Knöchelgelenkachse rechtwinkelig zu der Fußmittellinie in Draufsicht, also in der Projektion in eine horizontale Ebene, ist die Gelenkachse in anteriore Richtung zu der natürlichen Knöchelgelenksachse geneigt. Dies kann durch eine entsprechende schräge Kröpfung der Fußstrebe erreicht werden, so dass der proximale Endbereich, an dem das Gelenk angeordnet ist, um den gewünschten Winkel verdreht wird. In Draufsicht ist die Gelenkachse entgegen dem Uhrzeigersinn um den gewünschten Winkel verdreht, bevorzugt zwischen 5° und 20° um die Längserstreckung der Fußstrebe nach vorn verdreht, insbesondere zwischen 7° und 15°, als Kompromisswinkel um 11°, so dass die Fußmittelachse oder Fußlängsachse zu der Laufrichtung nach außen rotiert ist.

Bevorzugt ist das Gelenk im angelegten Zustand auf Höhe des Knöchelvorsprunges angeordnet, bei einer medialen Orientierung der Fußstrebe auf Höhe des medialen Knöchelvorsprunges, bei einer lateralen Anordnung der Fußstrebe auf Höhe des lateralen Knöchelvorsprunges. Die Gelenkachse befindet sich im angelegten Zustand im Wesentlichen auf Höhe des Kompromissdrehpunktes. Ein Ausgleich in der Höhe und Orientierung entweder durch eine Verformung der Fußstrebe oder aber durch ein Auswechseln der Winkel oder aber durch Einsatz von Distanzelementen erfolgen kann. Der Kompromissdrehpunkt im Knöchelgelenk ist der mechanische Drehpunkt des natürlichen Knöchelgelenks, der unterhalb des höchsten knöchernen Punktes des medialen Knöchelgelenkes auf Höhe des unteren Endes der Tibia liegt. Von dort verläuft die Knöchelgelenkachse horizontal in Richtung lateral in einem Winkel von 90° zu der Bewegungsrichtung.

Eine besonders unauffällige Art und Weise des Tragens der Orthese ist dann gegeben, wenn die Fußstrebe medial angeordnet ist, da die mediale Anordnung der Unterschenkelschiene sehr unauffällig ist und darüber hinaus die knöcherne Struktur des Fußes im Bereich des Sprungbeins und des Fersenbeins eine gute Abstützung der Fußstrebe ermöglicht.

Die Fußstrebe kann sich winkelförmig unter den Fuß erstrecken und eine Bodenstrebe aufweisen, die entweder angeformt oder daran befestigt ist. Damit erstreckt sich die Bodenstrebe unterhalb des Fußes in, auf oder unter der Fußplatte und ermöglicht eine sichere Verbindung der Fußplatte mit der Fußstrebe.

Neben einer separaten Ausgestaltung der Fußplatte und der Fußstrebe und einer Befestigung aneinander ist es grundsätzlich auch möglich, dass die Fußstrebe und die Fußplatte einstückig ausgebildet sind, hierzu ist es notwendig, die jeweils geeigneten Materialien einzusetzen. Neben einer Ausgestaltung aus einem Metall ist es möglich, die Fußstrebe und die Fußplatte einteilig aus einem Verbundwerkstoff, insbesondere einem faserverstärktem Kunststoff herzustellen, wobei eine Aufnahme für das Gelenk oder eine Gelenkeinrichtung angeformt oder aus einem anderen Material ausgebildet sein kann.

Zur Erhöhung des Tragekomforts können die Fußplatte und/oder die Fußstrebe zumindest teilweise mit einer Ummantelung versehen sein, wodurch der Tragekomfort verbessert werden kann. Darüber hinaus können federnde Eigenschaften der Ummantelung zur Verbesserung des Abrollverhaltens genutzt werden.

Nachfolgend werden Ausführungsbeispiele der Erfindung anhand der beigefügten Figuren näher erläutert. Es zeigen:
- Figur 1 -: eine Orthese als AFO in perspektivischer Gesamtansicht;
- Figur 2 -: ein Fußteil der Orthese ohne Unterschenkelschiene in Seitenansicht;
- Figur 3 -: ein Fußteil in Untenansicht;
- Figur 4 -: ein Fußteil in Draufsicht;
- Figur 5 -: ein Fußteil in perspektivischer Schrägansicht;
- Figur 6 -: ein Fußteil in Rückansicht; sowie
- Figur 7 -: eine schematische Darstellung eines Fußes.

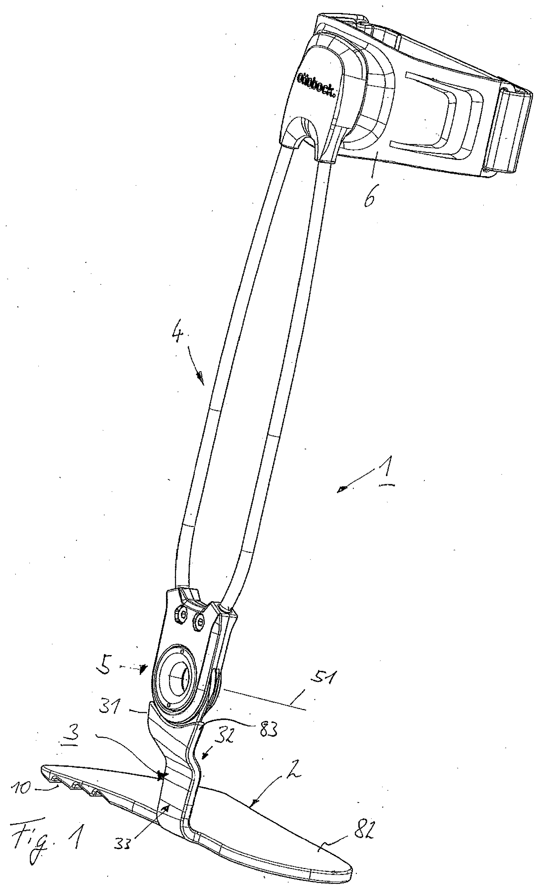

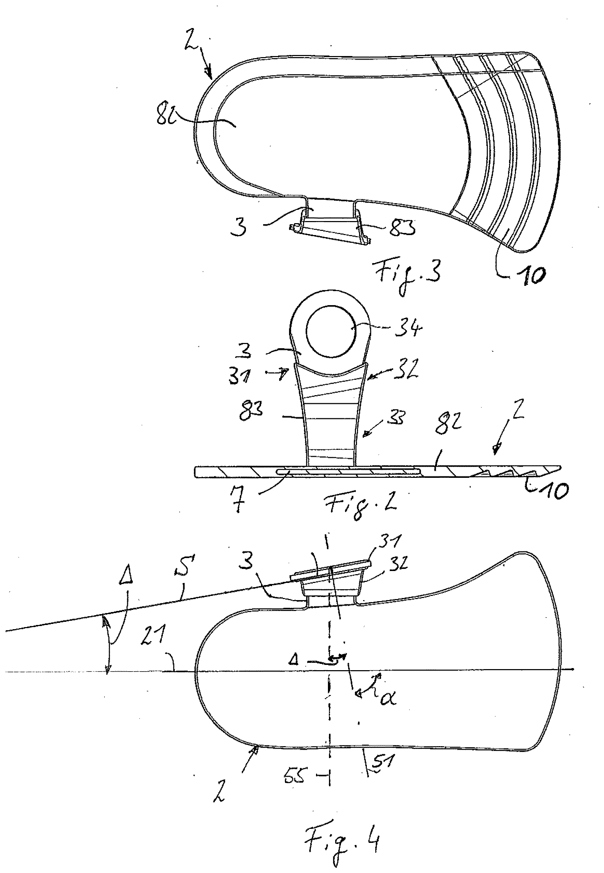

Figur 1 zeigt eine Orthese 1 in Gestalt einer Knöchel-Fuß-Orthese mit einer Fußplatte 2 zur Auflage eines nicht dargestellten Fußes, von der in proximaler Richtung eine Fußstrebe 3 absteht. Die Fußstrebe 3 ist an ihrem distalen Endbereich 33 an der Fußplatte 2 befestigt oder einstückig damit ausgebildet. Die Befestigung der Fußstrebe 3 kann durch Einlaminieren, separate Befestigungselemente wie Schrauben oder Nieten, Einkleben, Einrasten oder andere formschlüssige oder stoffschlüssige Befestigungsarten erfolgen. Ebenso ist es möglich, dass an der Fußstrebe 3 eine sich in der Ebene der Bodenplatte 2 erstreckende Bodenstrebe 7, die in der Figur 2 gezeigt ist, angeformt oder befestigt ist, die sich in, auf oder unter der Fußplatte 2 erstreckt und somit einen Winkel bildet, der eine starre, gelenklose Anbindung der Fußstrebe 3 an der Fußplatte 2 ermöglicht.

An dem proximalen Endbereich 31 der Fußstrebe 3 ist ein Gelenk 5 befestigt, das als flaches Gelenk ausgebildet ist und über das eine Unterschenkelschiene 4, an dessen proximalen Ende eine Befestigungseinrichtung 6 zur Festlegung an einem nicht dargestellten Unterschenkel angeordnet ist, schwenkbar an der Fußstrebe 3 befestigt ist. Die Befestigungseinrichtung 6 umschließt den Unterschenkel vollumfänglich und weist eine Manschette mit einem Schließgurt auf. Über zwei gebogene Streben der Unterschenkelschiene 4 ist die Neigung des oberen Anlagepunktes der Unterschenkelschiene 4 relativ zu dem Unterschenkel bzw. relativ zu der Orientierung der Fußstrebe 3 einstellbar. Neben der dargestellten Orthese zur Korrektur einer Beinfehlstellung kann die Fußstrebe 3 gegenüber der Unterschenkelschiene 4 federnd vorgespannt sein, beispielsweise über eine in dem Gelenk 5 angeordnete Feder, um eine Fußheberorthese bereitzustellen.

Die Unterschenkelschiene 4 ist über das Gelenk 5 relativ zu der Fußstrebe 3 um eine Gelenkachse 51 verschwenkbar, eine gelenkige Verschwenkung um eine andere Achse ist nicht vorgesehen.

Die Fußplatte 2 weist eine Ummantelung 82 auf, die aus einem flexiblen, gegebenenfalls elastischen Werkstoff bestehen kann. An der Unterseite der Fußplatte 2 sind im dargestellten Ausführungsbeispiel drei hintereinander angeordnete Schwächungen 10 ausgebildet, entlang der die Fußplatte 2 einfach kürzbar ist.

Auch an der Fußstrebe 3 ist eine Ummantelung 83 angeordnet, die sich bis zum proximalen Ende 31 der Fußstrebe 3 erstreckt und lediglich die Gelenkeinrichtung 5 und den angrenzenden Bereich des proximalen Endbereiches 31 freilässt, so dass eine freie Verschwenkung der Unterschenkelschiene relativ zu der Fußstrebe 3 nicht behindert wird. Die Ummantelung 82, 83 verbessert den Tragekomfort, da die gegebenenfalls scharfkantigen Werkstoffe der Fußstrebe 3 und/oder der Fußplatte 2 dadurch nicht in direkten Kontakt mit dem Körper des Orthesennutzers kommen.

An der Fußstrebe 3 ist im proximalen Endbereich 31 ein Absatz 32 ausgebildet, der von der Fußplatte 2 wegweist, so dass der proximale Endbereich 31 der Fußstrebe 3 versetzt und weiter entfernt von der Fußplatte 2 als der distale Bereich 33 der Fußstrebe 3 ist. Über den Absatz 32 wird der Bereich, in dem oder an dem das Gelenk 5 angeordnet ist, von der Fußplatte 2 entfernt, so dass bei einer medialen Anordnung der Fußstrebe 3 der proximale Endbereich 31 medial versetzt zu der Fußplatte 2 befindlich ist. Bei einer lateralen Anordnung der Fußstrebe 3 erfolgt über den Absatz 32 ein Versatz in lateraler Richtung, also von dem Fuß weg. Durch den Absatz 32 und die dadurch vergrößerte Entfernung des proximalen Endbereiches 31 der Fußstrebe 3 im Vergleich zu einem distalen Endbereich 33 der Fußstrebe, beispielsweise im Bereich des Überganges von der Fußplatte 2 zur Fußstrebe 3, ist es möglich, dass die Fußstrebe 3 sehr nah an der knöchernen Struktur des Fußes entlanggeführt werden kann, ohne dass das Gelenk 5 im Bereich des Knöchels an dem knöchernen Knöchelvorsprung scheuert oder unangenehm anliegt.

Die Fußstrebe 3 weist in dem Ausführungsbeispiel einen distalen Endbereich 33 auf, der sich im Wesentlichen geradlinig von der Fußplatte 2 weg erstreckt, der distale Endbereich 33 und der proximale Endbereich 31 sind durch den Absatz 32 hinsichtlich einer Sagittalebene versetzt zueinander. Zumindest der distale Endbereich 33 der Fußstrebe 3 ist als Flachmaterial ausgebildet, im dargestellten Ausführungsbeispiel ist die gesamte Fußstrebe 3 mit distalem Endbereich 33, Absatz 32 und proximalem Endbereich 31 aus einem Flachmaterial, insbesondere einem Metall ausgebildet.

Figur 2 zeigt das Fußteil der Orthese ohne die Unterschenkenschiene 4 und ohne die Gelenkeinrichtung mit der Fußplatte 2, die eine im Wesentlichen ebene Oberseite und eine Unterseite mit Schwächungen 10 im vorderen Bereich aufweist. Die Schwächungen 10 sind sägezahnförmig ausgebildet und bilden Sollschnittstellen, entlang derer eine Kürzung der Fußplatte 2 und damit eine Anpassung an verschiedene Schuhformen oder Fußformen erfolgen kann. Die Schwächungen 10 weisen eine Schräge auf, die von vorn nach hinten in einer schräg nach unten geneigten Richtung verlaufen, so dass ein Abrollen erleichtert wird. Sofern die Schräge in einer Spitze ausläuft, ist an der Vorderkante der Fußplatte 2 kein Absatz ausgebildet. Nach Möglichkeit wird ein Absatz von kleiner 2 mm, vorzugsweise kleiner 0,5 mm angestrebt, um auch im empfindlichen Fußbereich keine störenden Einflüsse beim Abrollen zu erzeugen. In der seitlichen Schnittdarstellung gemäß Figur 2 ist die Bodenstrebe 7 zu erkennen, die im dargestellten Ausführungsbeispiel mit der Fußstrebe 3 ausgebildet ist und ein gelenkloses Winkelstück darstellt. Die Bodenstrebe 7 ist mit einer Ummantelung 82 versehen, so dass aus der Bodenstrebe 7 und der Ummantelung 82 die Fußplatte 2 gebildet wird. Die Ummantelung 82 erstreckt sich auch über einen Teil der Fußstrebe 3 und ist dort mit dem Bezugszeichen 83 versehen.

Der distale Abschnitt 33 der Fußstrebe 3 erstreckt sich im Wesentlichen senkrecht nach oben von der Fußplatte 2, daran schließt sich ein Absatz 32 an, in dem das Material der Fußstrebe 3 zunächst nach außen und dann nach oben gebogen ist, so dass im proximalen Endbereich 31 eine Gelenkeinrichtung in einer Aufnahme 34, die im dargestellten Ausführungsbeispiel als runde Ausnehmung ausgebildet ist, oder das Lager für die schwenkbare Befestigung der Unterschenkelschiene 4 angeordnet werden kann. Der proximale Endbereich 31 ist flächig ausgebildet und verläuft im Wesentlichen in einer Schwenkebene, die durch die Gelenkachse 51 definiert wird. Die Schwenkebene steht orthogonal zu der Gelenkachse 51 und gibt die Schwenkrichtung S des Gelenks vor. Die Hauptebene des proximalen 31 Endbereiches liegt in der Schwenkebene oder parallel dazu. Die Schwenkebene ist im Wesentlichen in der normalen Laufrichtung L orientiert.

Figur 3 zeigt das Fußteil gemäß Figur 2 in Untenansicht, die Schwächungen 10, im Ausführungsbeispiel drei Schwächungen 10, verlaufen korrespondierend zu der Vorderkante der Fußplatte 2, die Fußstrebe 3 ist aus der Fußplatte 2 herausgebogen.

Figur 4 zeigt eine Draufsicht auf das Fußteil mit der Fußplatte 2 und der senkrecht aus der Blattebene herausstehenden Fußstrebe 3. Die Fußstrebe 3 ist, wie bei allen anderen dargestellten Ausführungsformen, medial angeordnet, ungefähr im Bereich des knöchernen Knöchelvorsprunges des Schienbeins, und erstreckt sich senkrecht nach oben. Es ist ein minimaler Biegeradius vorgesehen, mit dem die Fußstrebe 3 über den distalen Bereich 33 mit der Fußplatte 2 verbunden ist, so dass der distale Bereich 33 der Fußstreber 3 möglichst nah an der knöchernen Struktur des Fußes anliegen kann.

Der Absatz 32 ist asymmetrisch ausgebildet. Das vordere Ende des Absatzes 32 ist weiter nach medial gebogen als das hintere Ende, so dass sich in Draufsicht eine Verschwenkung oder eine winklige Orientierung des distalen Endbereiches 31 relativ zu der angedeuteten natürlichen Knöchelgelenksachse 55 und relativ zu einer Fußmittelachse oder einer Fußmittellinie 21 der Fußplatte 2 ergibt. Die winkelige Orientierung weicht von einem rechten Winkel ab. Die Gelenkachse 51 des Gelenks 5 ist in einem Winkel Δ zu der natürlichen Gelenkachse orientiert ausgerichtet und in einer horizontalen Ebene nach vorn, also in anteriorer Richtung verkippt. Der Winkel Δ zwischen der natürlichen Gelenksache 55 und der Gelenkachse 51 der Gelenkeinrichtung 5 beträgt zwischen 5° und 20°, bevorzugt zwischen 7° und 15°, insbesondere 12°, um eine Außenrotation beim Gehen oder Stehen zu kompensieren. Dementsprechend beträgt der Winkel Δ zwischen der Schwenkrichtung S und der Fußmittellinie 21 zwischen 5° und 20°, bevorzugt zwischen 7° und 15°, insbesondere 12°. Dadurch ist die Gelenkachse 51 in der Horizontalebene gesehen geneigt zu der Mittellinie 21 der Fußplatte 2, die im Wesentlichen der Mittellinie eines Fußes entspricht und von dem mittleren Fersenbereich senkrecht zu der Knöchelgelenkachse 55 in anterior-posterior-Richtung verläuft, orientiert. Der Winkel α der Gelenkachse 51 zur Fußmittellinie 21 beträgt somit zwischen 85° und 70° und ist dabei nach anterior gerichtet, also nach vorn verdreht orientiert. Die Fußmittellinie 21 ist relativ zu der Laufrichtung L, die der Schwenkrichtung S entspricht, in einem Winkel zwischen 20° und 5° nach außen rotiert.

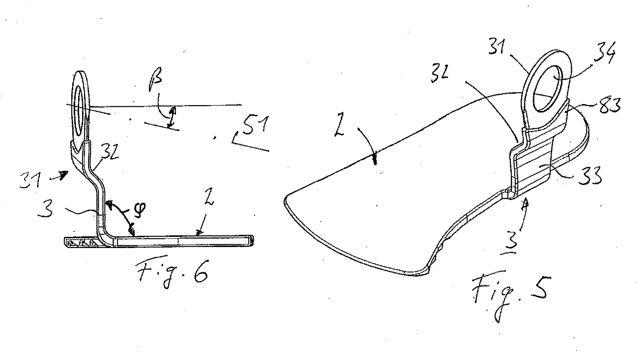

Figur 5 zeigt das Fußteil in einer perspektivischen Schrägdraufsicht, in der zu erkennen ist, dass die Fußstrebe 3 mit dem distalen Abschnitt 33 im Wesentlichen senkrecht von der Fußplatte 2 absteht. Der Absatz 32 führt dazu, dass der proximale Endbereich 31 mit der Ausnehmung 34 als Befestigungsaufnahme für die Gelenkeinrichtung 5 ebenfalls senkrecht nach oben orientiert ist, allerdings in Längserstreckung der Fußstrebe 3 nach vorn oder in anterior-Richtung verdreht.

Figur 6 zeigt das Fußteil in Hintenansicht, der Winkel ϕ zwischen der Fußplatte 2 und dem distalen Bereich der Fußstrebe 3 beträgt im dargestellten Ausführungsbeispiel 90°, was bei einer medialen Anordnung der Fußstrebe 3 zu einer guten Anlage an dem Fußknochen führt. Eine individuelle Anpassung kann durch eine plastische Verformung der Fußstrebe 3 relativ zu der Fußplatte 2 erfolgen, so dass die Fußstrebe 3 möglichst nahe an dem Fuß anliegt. Der distale Endbereich 31 der Fußstrebe 3 kann neben einer Verdrehung um die Längserstreckung in anteriorer Richtung ebenfalls eine Neigung aufweisen, so dass sich ein Winkel β zwischen der Gelenkachse 51 und der dargestellten Horizontalen ergibt. Dadurch wird es ermöglicht, dass die Gelenkachse 51 des nicht dargestellten Gelenkes individuell anpassbar ist. Die Gelenkachse 51 ist bevorzugt so positioniert, dass sie mit der natürlichen Knöchelgelenkachse zusammenfällt. Eine Höheneinstellung kann sich durch eine Winkeleinstellung der Fußstrebe 3, durch eine Änderung des Absatzes 32, durch Distanzelemente oder durch Einsätze oder Aufsätze bei der Befestigung der Unterschenkelschiene 4 ergeben. Die Gelenkachse 51 ist im angelegten Zustand ungefähr, bevorzugt genau auf der Höhe des Kompromissdrehpunktes des natürlichen Knöchelgelenks positioniert.

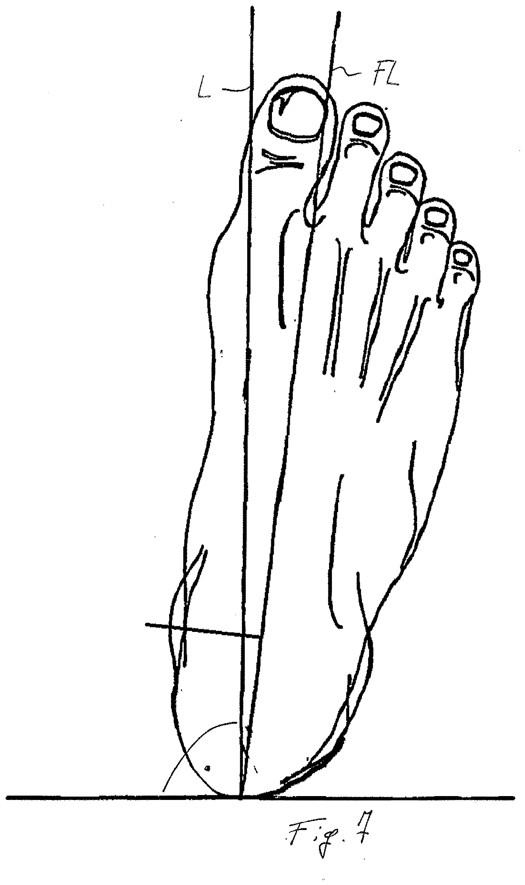

Figur 7 zeigt eine schematische Darstellung eines Fußes, in der die Laufrichtung L und die Fußlängsachse FL eingezeichnet sind. Bei einer normalen Fußstellung fallen die Laufrichtung L und die Fußlängsachse FL auseinander, in der Regel die die Fußlängsachse FL gegenüber der Laufrichtung L außenrotiert orientiert.

## Patentansprüche

1. Orthese mit einer Fußplatte (2) zur Auflage eines Fußes, mit einer davon in proximaler Richtung abstehenden Fußstrebe (3) und einer an der Fußstrebe (3) befestigten Unterschenkelschiene (4), die über ein Gelenk (5) um eine Gelenkachse (51) relativ zu der Fußstrebe (3) schwenkbar daran gelagert ist, **dadurch gekennzeichnet, dass** an dem proximalen Endbereich (31) der Fußstrebe (3) ein Absatz (32) in die von der Fußplatte (2) abgewandten Richtung angeordnet oder ausgebildet ist, sowie dass der proximale Endbereich (31) und die Gelenkachse (51) zu der Längserstreckung der Fußstrebe (3) tordiert orientiert sind.

2. Orthese nach Anspruch 1, **dadurch gekennzeichnet, dass** der Absatz (32) durch eine Kröpfung in der Fußstrebe (3) oder ein an der Fußstrebe (3) befestigtes Winkelstück ausgebildet ist.

3. Orthese nach einem der voranstehenden Ansprüche, **dadurch gekennzeichnet, dass** die Fußstrebe (3) oder das Winkelstück aus einem starren Material ausgebildet ist.

4. Orthese nach einem der voranstehenden Ansprüche, **dadurch gekennzeichnet, dass** die Fußstrebe (3) sich in einem Winkel ϕ zwischen 80° und 100° von der Fußplatte (2) in proximaler Richtung erstreckt.

5. Orthese nach einem der voranstehenden Ansprüche, **dadurch gekennzeichnet, dass** die Fußstrebe (3) aus einem Flachmaterial ausgebildet ist.

6. Orthese nach einem der voranstehenden Ansprüche, **dadurch gekennzeichnet, dass** die Gelenkachse (51) in medial-lateral Richtung in distale Richtung in einem Winkel β geneigt ist.

7. Orthese nach einem der voranstehenden Ansprüche, **dadurch gekennzeichnet, dass** die Gelenkachse (51) in einem Winkel α schräg zu der Fußmittellinie (21) der Fußplatte (2) verläuft.

8. Orthese nach Anspruch 7, **dadurch gekennzeichnet, dass** im angelegten Zustand die Gelenkachse (51) eine Schwenkrichtung (S) definiert, die in einem Winkel Δ zwischen 5° und 20° zu der Fußlängsachse (FL) oder der Fußmittellinie (21) der Fußplatte (2) orientiert ist.

9. Orthese nach einem der voranstehenden Ansprüche, **dadurch gekennzeichnet, dass** das Gelenk (5) im angelegten Zustand im Wesentlichen auf Höhe eines Kompromissdrehpunktes angeordnet ist.

10. Orthese nach einem der voranstehenden Ansprüche, **dadurch gekennzeichnet, dass** die Fußstrebe (3) medial an der Fußplatte (2) angeordnet ist.

11. Orthese nach einem der voranstehenden Ansprüche, **dadurch gekennzeichnet, dass** an der Fußstrebe (3) eine Bodenstrebe (7) angeformt oder befestigt ist, die sich in oder unter die Fußplatte (2) erstreckt.

12. Orthese nach einem der voranstehenden Ansprüche, **dadurch gekennzeichnet, dass** die Fußstrebe (3) und die Fußplatte (2) einstückig ausgebildet sind.

13. Orthese nach einem der voranstehenden Ansprüche, **dadurch gekennzeichnet, dass** die Fußplatte (2) und/oder die Fußstrebe (3) zumindest teilweise mit einer Ummantelung (82, 83) versehen sind.

## Claims

1. Orthosis comprising a foot plate (2) for supporting a foot, a foot strut (3) projecting therefrom in the proximal direction, and a lower-leg rail (4) secured to said foot strut (3) and mounted thereupon by a joint (5) so as to pivot about a joint axis (51) relative to the foot strut (3), **characterized in that** a shoulder (32) is arranged or formed at the proximal end region (31) of the foot strut (3), in the direction facing away from the foot plate (2), and that the proximal end portion (31) and the joint axis (51) are twistingly oriented to the longitudinal extension of the foot strut (3).

2. Orthosis according to claim 1, **characterized in that** the shoulder (32) is formed by an offset in the foot strut (3) or an angle piece fastened to the foot strut (3).

3. Orthosis according to one of the previous claims, **characterized in that** the foot strut (3) or the angle piece are formed from a rigid material.

4. Orthosis according to one of the previous claims, **characterized in that** the foot strut (3) extends from the foot plate (2) in proximal direction at an angle ϕ between 80° and 100°.

5. Orthosis according to one of the previous claims, **characterized in that** the foot strut (3) is formed from a flat material.

6. Orthosis according to one of the previous claims, **characterized in that** the j oint axis (51) in medial-lateral direction is slanted at an angle β in distal direction.

7. Orthosis according to one of the previous claims, **characterized in that** the j oint axis (51) runs obliquely to the foot center line (21) of the foot plate (2) at an angle α.

8. Orthosis according to claim 7, **characterized in that** in the applied state, the joint axis (51) defines a swivel direction (S) which is oriented at an angle Δ between 5° and 20° to the foot longitudinal axis (FL) or the foot center line (21) of the foot plate (2).

9. Orthosis according to one of the previous claims, **characterized in that** in the applied state, the joint (5) is arranged essentially at the height of a compromise pivot point.

10. Orthosis according to one of the previous claims, **characterized in that** the foot strut (3) is arranged medially on the foot plate (2).

11. Orthosis according to one of the previous claims, **characterized in that** a floor strut (7) is molded or fastened to the foot strut (3) and extends into or below the foot plate (2).

12. Orthosis according to one of the previous claims, **characterized in that** the foot strut (3) is integral with the foot plate (2).

13. Orthosis according to one of the previous claims, **characterized in that** the foot plate (2) and/or the foot strut (3) are provided, at least to some extent, with a sheathing (82, 83).

## Revendications

1. Orthèse comprenant une plaque repose-pied (2) pour supporter un pied, une entretoise de soutien de pied (3), dépassant de ladite plaque dans la direction proximale, et une éclisse de bas de jambe (4) fixée à l'entretoise de soutien de pied (3) et montée sur celle-ci de manière à pouvoir pivoter par rapport à l'entretoise de soutien de pied (3) autour d'un axe d'articulation (51) par l'intermédiaire d'une articulation (5),
**caractérisée en ce qu'**un épaulement (32) est disposé ou formé dans la zone d'extrémité proximale (31) de l'entretoise de soutien de pied (3) dans la direction détournée de la plaque repose-pied (2),
et **en ce que** la zone d'extrémité proximale (31) et l'axe d'articulation (51) sont orientés en torsion par rapport à l'extension longitudinale de l'entretoise de soutien de pied (3).

2. Orthèse selon la revendication 1,
**caractérisée en ce que** l'épaulement (32) est formé par un coude dans l'entretoise de soutien de pied (3) ou par une pièce coudée fixée à l'entretoise de soutien de pied (3).

3. Orthèse selon l'une des revendications précédentes,
**caractérisée en ce que** l'entretoise de soutien de pied (3) ou la pièce coudée est réalisée en un matériau rigide.

4. Orthèse selon l'une des revendications précédentes,
**caractérisée en ce que** l'entretoise de soutien de pied (3) s'étend selon un angle ϕ compris entre 80° et 100° à partir de la plaque repose-pied (2) dans la direction proximale.

5. Orthèse selon l'une des revendications précédentes,
**caractérisée en ce que** l'entretoise de soutien de pied (3) est réalisée en un matériau plat.

6. Orthèse selon l'une des revendications précédentes,
**caractérisée en ce que** dans la direction médiale-latérale, l'axe d'articulation (51) est incliné selon un angle β en direction distale.

7. Orthèse selon l'une des revendications précédentes,
**caractérisée en ce que** l'axe d'articulation (51) s'étend selon un angle α en oblique par rapport à la ligne médiane de pied (21) de la plaque repose-pied (2).

8. Orthèse selon la revendication 7,
**caractérisée en ce que**, à l'état appliqué, l'axe d'articulation (51) définit une direction de pivotement (S) orientée selon un angle Δ compris entre 5° et 20° par rapport à l'axe longitudinal de pied (FL) ou à la ligne médiane de pied (21) de la plaque repose-pied (2).

9. Orthèse selon l'une des revendications précédentes,
**caractérisée en ce que**, à l'état appliqué, l'articulation (5) est située sensiblement au niveau d'un point de rotation de compromis.

10. Orthèse selon l'une des revendications précédentes,
**caractérisée en ce que** l'entretoise de soutien de pied (3) est disposée médialement sur la plaque repose-pied (2).

11. Orthèse selon l'une des revendications précédentes,
**caractérisée en ce qu'**une entretoise d'appui au sol (7) est conformée ou fixée sur l'entretoise de soutien de pied (3) et s'étend dans ou sous la plaque repose-pied (2).

12. Orthèse selon l'une des revendications précédentes,
**caractérisée en ce que** l'entretoise de soutien de pied (3) et la plaque repose-pied (2) sont réalisées d'un seul tenant.

13. Orthèse selon l'une des revendications précédentes,
**caractérisée en ce que** la plaque repose-pied (2) et/ou l'entretoise de soutien de pied (3) sont au moins partiellement pourvues d'un enrobage (82, 83).
